# EUROPÄISCHE PATENTSCHRIFT

(11) **EP 0 988 067 B1**
(45) Veröffentlichungstag und Bekanntmachung des Hinweises auf die Patenterteilung: **14.01.2004**
(21) Anmeldenummer: 97923706.2
(22) Anmeldetag: 10.06.1997
(51) Int. Cl.: A61L 27/00

(54) **BINÄRE TITAN-ZIRKON-LEGIERUNG FÜR CHIRURGISCHE IMPLANTATE SOWIE HERSTELLUNGSVERFAHREN**
BINARY TITANIUM-ZIRCONIUM ALLOY FOR SURGICAL IMPLANTS AND A SUITABLE MANUFACTURING PROCESS
ALLIAGE BINAIRE TITANE-ZIRCONIUM POUR IMPLANTS CHIRURGICAUX ET PROCEDE DE PRODUCTION CORRESPONDANT

(43) Veröffentlichungstag der Anmeldung: 29.03.2000
(73) Patentinhaber: INSTITUT STRAUMANN AG, 4437 Waldenburg (CH)
(72) Erfinder: STEINEMANN, Samuel, CH-1025 Saint-Sulpice (CH)
(74) Vertreter: Hepp, Dieter
(86) Internationale Anmeldenummer: PCT/CH1997/000230
(87) Internationale Veröffentlichungsnummer: WO 1997/029624

(56) Entgegenhaltungen:
- CH-A- 544 154
- US-A- 4 857 269
- MASSALSKI T. B.: "BINARY ALLOY PHASE DIAGRAMS Vol 2" 1986 , AMERICAN SOCIETY FOR METALS , OHIO US XP002055041 144030 siehe Seite 2142 - Seite 2143
- CHEMICAL ABSTRACTS, vol. 119, no. 10, 6.September 1993 Columbus, Ohio, US; abstract no. 103239, OKAZAKI, YOSHIMITSU ET AL: "Effect of alloying elements on mechanical properties of titanium alloys for medical implants" XP002055042 & NIPPON KINZOKU GAKKAISHI (1993), 57(3), 332-7 CODEN: NIKGAV;ISSN: 0021-4876, 1993,
- DATABASE METADEX MATERIALS INFORMATION, THE INSTITUTE OF METALS, LONDON, GB AMICK, D.D. (UNIVERSITY OF IDAHO): "An investigation of reaction forming by controlled passivation of titanium-zirconium alloys with niobium." XP002055043 & DISSERTATION ABSTRACTS INTERNATIONAL 55, (4), USA ISSN: 0419-4217,

## Beschreibung

### Anwendungsgebiet der Erfindung

Die vorliegende Erfindung betrifft eine Legierung aus Titan (Ti) und Zirkon (Zr) zur Herstellung von chirurgischen Implantaten. Eine erste Anwendung umfasst beispielsweise Schrauben, Platten und Nägel für die Frakturbehandlung und die operative Orthopädie sowie Endoprothesen. Eine zweite Anwendung umfasst die Zahnchirurgie mit enossalen Implantaten, Abutments und Elementen für Suprakonstruktionen. weiterhin ist die Legierung für Implantate zur Verankerung von Prothesen im Gesichtsbereich, also z.B. für maxillofaziale und extraorale Implantate, geeignet. Ferner betrifft die Erfindung ein Verfahren zur Herstellung einer solchen Legierung.

### Hintergrund der Erfindung und Stand der Technik

Die heute verwendeten metallischen Werkstoffe für chirurgische Implantate sind:
- rostfreier Stahl gemäss internationalem Standard ISO-Norm 5832-1;
- Kobalt-Chrom-Molybdän Legierungen gemäss ISO 5832-4, 5832-5, 5832-6;
- unlegiertes Titan gemäss ISO 5832-2;
- Legierungen: Titan-Aluminium-Vanadium (Ti6Al4V) gemäss ISO 5832-3; Titan-Aluminium-Eisen (Ti5Al2, 5Fe) gemäss ISO 832-10; Titan-Aluminium-Niob (Ti6Al7Nb) gemäss ISO 5832-11.

Diese Werkstoffe werden als gewebeverträglich angesehen im Sinne der gängigen Definition von "Biocompatibility: The ability of a material to perform with appropriate host response in a specific situation" (vgl. D.F. Williams: in Biomaterials: Proceedings of a Consensus Conference of the European Society for Biomaterials. Elsevier Science Publ. Amsterdam, 1989). Der Fremdkörper ruft im lebenden Gewebe nur eine minimale Reaktion hervor. Im Kontaktgewebe zum Metall wird Sequestrierung, als Einkapseln ohne pathologische Zellformen, oder Trägheit, erkennbar als lockeres Bindegewebe, beobachtet (vgl. S.G. Steinemann: Corrosion of Titanium and Titanium Alloys for Surgical Implants. Titanium, Science and Technology, Proc. 5th Int. Conf. Titanium. [G. Lütjering, U. Zwicker, W. Bunk, eds.], Deutsche Ges. Metallk. Oberursel, 1985, 1373).

Für Zahnimplantate, welche im Knochen verankert sind, ist eine stärkere Form von Gewebeverträglichkeit, nämlich Osseointegration, wirksam. Nach P.-I. Bränemark, G.A. Zarb, T. Albrektsson [editors]: Tissue-Integrated Prostheses. Quintessenz Publ. Chicago, 1985, p. 11, heisst es: "Osseointegration is defined as a direct structural and functional connection between ordered, living bone and the surface of a load-carrying implant". Diese Reaktion bedingt eine Wechselwirkung zwischen dem Fremdkörper und dem lebenden Gewebe, also mehr als nur träges Verhalten des Metalls. Zahnimplantate werden aus Titan hergestellt. Für dieses Metall wird im Tierversuch die vollständige Verankerung, nämlich die kraftschlüssige Verbindung gegen die Wirkung von Druck, von Scherung und von Zug gefunden (vgl. S.G. Steinemann, F. Straumann: Ankylotische Verankerung von Implantaten. Schweiz. Mschr. Zahnmed. 94, 1984, 682). Die vollständige Verankerung bedeutet auch Adhäsion, welche Vorgängen von Chemisorption zugeschrieben wird (vgl. S.G. Steinemann, J. Eulenberger, P.-A. Mäusli, A. Schroeder: Adhesion of bone to titanium. Biological and Biomechanical Performance of Biomaterials [P. Christel, A. Meunier, AJ.C. Lee, eds.], Elsevier Sci. Publ. Amsterdam, 1986, 409).

Implantate der Knochen- und Zahnchirurgie sind hohen Beanspruchungen ausgesetzt und müssen mechanisch fest sein. Wichtige Kenngrössen sind die Fliessgrenze, die Dehnung bis zum Bruch und das Elastizitätsmodul. Rostfreier Stahl und unlegiertes Titan haben niedrige Festigkeit und werden deshalb kaltverformt. Für Titan steigt damit die Zugfestigkeit von etwa 500 MPa auf etwa 700 MPa an. Die Festigkeit der standardisierten zwei Titanlegierungen von 900 - 1000 MPa wird aber nicht erreicht.

Ein Experiment, um Osseointegration zu messen, ist der Ausstossversuch. Dazu wird im Tierversuch ein zylindrisches Implantat im Knochen eingesetzt und die Ausstosskraft in Abhängigkeit der Liegezeit, typischerweise nach 12 Wochen, bestimmt. In solchen Experimenten wurde gefunden, dass die Verankerung von mechanisch aufgerauhtem, unlegiertem Titan, Ti6Al4v und Ti6Al7Nb von gleicher Stärke ist, dass aber eine mechanisch aufgerauhte und chemisch geätzte Oberfläche für Implantate aus Reintitan viel höhere Ausstosskräfte ergibt als eine gleiche Oberflächenbehandlung für Implantate aus Legierung (vgl. M. Wong, J. Eulenberger, R. Schenk, E. Hunziker: Effect of surface topology on the osseointegration of implant materials in trabecular bone. J. Biomed. Mater. Res. 29, 1995, 1569). Eines oder alle der drei zulegierten Elemente Al, V, Nb hemmen offenbar die Integration im Knochen.

Reintitan und Titanlegierungen können lokalen Angriff in engen Spalten zeigen, wenn sie heissen, Chlorid enthaltenden Elektrolyten ausgesetzt sind. Handbücher nennen Temperaturen über 70°C (vgl. B. Craig: Technical Note Corrosion. in Materials Properties Handbook: Titanium Alloys [R. Boyer, G. Welsch, E.W. Collings, eds.], ASM Int., Metals Park OH, 1994, 1065). Es gibt jedoch klinische Literatur, welche über die Korrosion im Spalt der Konus-Verbindung von künstlichen Hüftgelenken und am Schaft solcher Prothesen im Zementbett berichtet. Die bekannten Titanwerkstoffe für Implantate sind offenbar nicht beständig gegen Spaltkorrosion.

Die CH-A-544 154 nannte eine binäre Titan-Zirkon Legierung mit 25 - 75 Gewichts-% Zr für Implantate. Der Zusammensetzungsbereich entspricht hohen mechanischen Festigkeiten. Diese Metalle sind reaktiv, zeigen exotherme Oxydation und sind deswegen nicht einfach zu verarbeiten. Andererseits entspricht die Legierungsformel einer Atomkonzentration von 15 - 61% Zr, d. h. das zulegierte Element hat auf elektrochemische und biologische Reaktionen starken Einfluss. Das ist nicht erwünscht, und die für Titan spezifischen Reaktionen im lebenden Gewebe sollen nicht verloren gehen.

### Aufgabe der Erfindung

Angesichts des bisherigen Fehlens einer optimalen Legierung für die obengenannten Verwendungsgebiete liegt der Erfindung das Problem zugrunde, ein metallisches Biomaterial zu schaffen, welches erstens eine hohe Festigkeit aufweist, zweitens in Weich- sowie Hartgewebe verträglich ist und integriert wird und drittens beständig gegen Spaltkorrosion ist.

### Wesen der Erfindung

Vorgeschlagen wird eine binäre Titan-Zirkon-Legierung, enthaltend die beiden Legierungsbestandteile Titan und Zirkon und gegebenenfalls bis zu 0.5 Gewichts-% Hafnium (als Verunreinigung von Zirkonium), dadurch gekennzeichnet, dass diese Legierung (i) eine binäre Legierung mit einem Anteil an Zirkon von weniger als 25 Gewichts-%, aber mehr als 5 Gewichts-% darstellt, (ii) 0.1 bis 0.3 Gewichts-% Sauerstoff als festigkeitssteigernder Zusatz, enthält und (iii) bis maximal 1 Gewichts-% festigkeitssteigernde Zusätze und technische Verunreinigungen aufweist, wobei (iv) die Komponenten Titan, Zirkon, sowie die gegebenenfalls anwesenden festigkeitssteigernden Zusätze und technische Verunreinigungen zusammen 100 Gewichts-% ergeben, Vorzugsbereiche des Zirkonanteils liegen zwischen 19 Gewichts-% und 10 Gewichts-%.

Die Charakteristika des Herstellungsverfahrens der Legierung bestehen darin, dass die Legierung warm geschmiedet und danach kaltverformt wird. Der Schmiedevorgang wird bei Temperaturen über 850°C, mit anschliessender schneller Abkühlung der Legierung, ausgeführt. Alternativ kann der Schmiedevorgang im Bereich der alpha/beta Phasenumwandlung bei 770°C bis 830°C ausgeführt werden, um besondere Gefügestrukturen zu erzeugen.

Dank der Erfindung steht nun ein metallisches Biomaterial für das definierte Anwendungsgebiet zur Verfügung, das eine ausreichende Festigkeit'sowie ausgezeichnete Gewebeverträglichkeit in Weich- und Hartgewebe besitzt und überdies beständig gegen Spaltkorrosion ist.

### Detailbeschreibung

Es ist bekannt, dass die mechanischen Eigenschaften von Titan durch Zulegieren von Zirkon deutlich angehoben werden (vgl. A.G. Imgram, D.N. Williams, H.R. Ogden: Tensile properties of binary titanium-zirconium and titanium-hafnium alloys. J. Less-Common Metals 4, 1962, 217). Eigene Messungen an fünf Legierungen sind in *Tabelle 1* zusammengestellt. Die Legierungen wurden aus technisch reinen Metallen erschmolzen und enthielten neben Ti, Zr 0,09 - 0,11% O und 0,02 - 0,03% N; ihr Zustand war "geschmiedet oberhalb der alpha-beta Umwandlung und abgekühlt".

**Tabelle 1**

| Zusammensetzung | Zugfestigkeit | Fliessgrenze | Bruchdehnung | Bruchspannung |
|---|---|---|---|---|
| Gew.-% Zr | MPa | MPa | % | MPa |
| Ti + 5,1 | 496 | 429 | 23,1 | 1526 |
| + 9,9 | 558 | 503 | 18,1 | 1436 |
| + 14,9 | 655 | 559 | 14,5 | 1469 |
| + 19,9 | 748 | 728 | 10,9 | 1454 |
| + 25,6 | 779 | 670 | 8,5 | 1127 |

Es ist nicht bekannt, dass die binäre Titan-Zirkon Legierung durch Kaltverformen und/oder durch Zugabe von Sauerstoff weiter verfestigt werden kann. Die erfindungsgemässen Legierungen lassen sich auch gut kaltverformen, z.B. durch Walzen. An einer nach Warmschmieden oberhalb der alpha-beta Umwandlung geglühten Ti15Zr Legierung wurden die Festigkeitswerte gemäss *Tabelle 2* gefunden:

**Tabelle 2**

| Material Ti15Zr | Zugfestigkeit | Fliessgrenze | Vickers-Härte |
|---|---|---|---|
| | MPa | MPa | kg/mm² |
| nicht kalt verformt gewalzt 20% | 769 | 704 | 203 259 |
| 40% | 883 | 873 | 275 |
| 60% | 944 | 937 | 309 |

Sauerstoff ist als kontrollierte Legierungskomponente zu betrachten, welche die Festigkeitseigenschaften beeinflusst. Resultate sind in *Tabelle 3* zusammengestellt.

**Tabelle 3**

| Ti15Zr + Sauerstoff | Zugfestigkeit | Fliessgrenze | Vickers-Härte |
|---|---|---|---|
| | [MPa] | [MPa] | [kg/mm²] |
| 0,1% O | 655 | 559 | 203 |
| 0,2% O | 780 | 670 | 240 |
| 0,3% O | 864 | 735 | 267 |

Die mechanischen Eigenschaften der bekannten und heute verwendeten Titanmetalle ergeben sich aus Tabelle 4 (vgl. ASM Metals Handbook 9th edition, Vol. 3, Properties and Selection: Stainless Steels, Tool Materials and Special Purpose Metals. Amer. Soc. Metals, Metals Park OH, 1980, 353; S.G. Steinemann, P.-A. Mäusli, S. Szmukler-Moncler, M. Semlitsch, O. Pohler, H.-E. Hintermann, S.M. Perren: Beta-titanium alloy for surgical implants. Titanium 92 Science and Technology [F.H. Froes, I. Caplan, eds.], TMS Warrentale PA, 1993, 2689).

**Tabelle 4**

| Material | Zugfestigkeit | Fliessgrenze | Bruchdehnung | Bruchspannung |
|---|---|---|---|---|
| | MPa | MPa | % | MPa |
| Ti Nuance 2 | 450 | | | |
| Nuance 4 | 620 | 550 | 20,0 | |
| Nuance 4 | 785 | 692 | 18,3 | 1095 |
| kaltverformt | | | | |
| Ti6A14V | 1076 | 940 | 14,7 | 1429 |
| Ti6A17Nb | 1024 | 921 | 14,0 | 1400 |

Die Bruchspannung (Bruchlast/Querschnitt an Bruchstelle) ist ein Mass für die Zähigkeit eines Metalls. Sie ist bemerkenswert hoch für die einphasigen Ti-Zr-Legierungen und etwa gleich jener der zweiphasigen (alpha-beta) Legierungen Ti6Al4V und Ti6Al7Nb. Auch die Zugfestigkeit und Fliessgrenze des erfindungsgemässen Werkstoffs sind besser als jene von kaltverformtem, unlegiertem Titan der Nuance 4 und erreichen nahezu jene der alpha-beta Legierungen, wenn der Effekt von Kaltverformen und Sauerstoffzulegieren genutzt wird. Die Kaltverformung und Sauerstoffzugabe ändert übrigens in keiner Weise die Gewebeverträglichkeit der binären Ti-Zr Legierung.

Zellkulturen sind ein gängiger Test, um die Reaktionen in Gegenwart metallischer Fremdkörper zu kennen. Für Weichund Hartgewebe sind Experimente mit Fibroblast-, respektive Osteoblast-Zellen interessant. Drei solcher Experimente wurden angestellt, nämlich erstens Sprossung von Osteoblasten auf einer trägen Kunststoffunterlage, wobei die Metalloxyde im Kulturmedium gelöst sind (Konzentration etwa mikromolar, gesättigt), zweitens Sprossung von Fibroblasten auf dem Metall im Kulturmedium und drittens Sprossung von.

Osteoblasten auf dem Metall im Kulturmedium. Folgende Reaktionen wurden beobachtet (s. *Tabelle 5*):

**Tabelle 5**

| Test | Hemmung | keine Hemmung |
|---|---|---|
| Osteblasten im gelösten Metallsalz | | Al, Sn, Ti, Zr, Ta |
| Fibroblasten auf Metall | Cu, Mo, V | Ti, Nb, Zr, Ta |
| Osteoblasten auf Metall | Zn, Fe, Sn, Cu, Al, Mo, V, Ni, Ag, Nb, Ta | Ti, Zr |

Es wurde keine Reaktion von Osteoblasten auf die im Kulturmedium gelösten Salze gefunden, und die fünf geprüften Metalle haben auch im Implantationstest (s. Steinemann, a.a.O., 1985) keine toxische Wirkung. Das Resultat der Experimente mit Fibroblasten ist dasselbe wie der Implantationstest, wo sich die vier Metalle Ti, Nb, Zr, Ta träge verhalten und Cu, Mo, V toxisch sind oder Sequestrierung bewirken. Das Resultat der Experimente mit Osteoblast-Zellen, welche im Kontakt mit dem Metall sind, ist deutlich anders; alleine Ti und Zr verhalten sich träge. Das Wachstum von Osteoblast-Zellen und deren unbehinderte Vermehrung ist aber Voraussetzung der Entstehung von Knochen wie auch der Osseointegration.

Die Adhäsion von Weichgewebe und insbesondere auch von Knochen am Implantat bedeutet eine Reaktion von "Verkleben" im atomaren Massstab zwischen den organischen und mineralischen Stoffen und dem Metall. Organische Stoffe sind z. B. die Aminosäuren als Bausteine der Proteine und als Komponenten der sogenannten Grundsubstanz (organischer Anteil von Knochen). Calcium und Phosphate sind Hauptbestandteile des Knochenminerals. Man spricht von Adsorption und Chemisorption (Kürzel für chemische Adsorption). Es ist bekannt, dass Aminosäuren auf Titanoxyd (Anatas und Rutil in Pulverform) und auf Zirkonoxyd adsorbiert werden. Es ist auch bekannt, dass Aminosäuren auf an Luft oxydiertem Titanmetall chemisorbiert werden (vgl. J.M. Gold, M. Schmidt, S.G. Steinemann: XPS study of amino acid adsorption to titanium surfaces. Helv. Phys. Acta 62, 1989, 246; J.M. Gold, M. Schmidt, S.G. Steinemann: XPS study of retrieved titanium and Ti älloy implants. Clinical Implant Materials [G. Heimke, U. Soltesz, A.J.C. Lee, eds.], Elsevier Sci. Publ. Amsterdam, 1990, 69). Es ist nicht bekannt, ob und wie Aminosäuren auf Zirkon und Ti-Zr-Legierungen adsorbiert werden und in welcher Form Calcium und Phosphor vorkommen. Experimente mittels Photoelektronen-Spektroskopie ergaben die Resultate gemäss *Tabelle 6.*

**Tabelle 6**

| Material | Aminosäuren | Ca²⁺ | H₂PO₄⁻ und HPO₄²⁻ |
|---|---|---|---|
| Ti | ++ | ++ | ++ |
| Ti + 25 Gew.-% Zr | + | + | + |
| + 50 Gew.-% Zr | + | - | + |
| Zr | + | - | + |
| Legende: + adsorbiert ++ stark adsorbiert - keine Adsorption | | | |

Aminosäuren werden chemisorbiert. Calcium und Phosphate binden in Form von Oberflächenkomplexen mit dem oxydierten Metall. Die Reaktionen sind stark, besonders für Ti-reiche Legierungen, und sie betreffen alle drei Reaktanden der Osseointegration. Andererseits fehlt Adsorption oder ist schwächer für Zirkon-reiche Legierungen.

Legieren mit Zr hat auch die vorteilhafte Wirkung, den Korrosionswiderstand von Ti in Chlorid enthaltenden Elektrolyten zu erhöhen. Der Effekt verbessert die Beständigkeit gegen Spaltkorrosion, doch nur wenn der Anteil von Zr 5 Gewichts-% übersteigt.

Die Konzentration von 25 Gewichts-% Zr entspricht einer Partikelkonzentration von 14,9 Atom-%, oder 1 Zr-Atom auf etwa 7 Atome der Legierung. Dieses Verhältnis charakterisiert die Titan-reiche Legierung. Ist der Anteil 1 Zr/12 Atome im Metall, entsprechend 8,3 Atom-% (= 14,6 Gewichts-%), so nennt man die Legierung verdünnt und die Titankomponente dominiert dann für die Eigenschaften des Metalls, insbesondere auch für die Verträglichkeit und die Oberflächenreaktionen. Eine bevorzugte Zusammensetzung ist deswegen der Bereich von weniger als 19 Gewichts-% aber mehr als 10 Gewichts-% Zirkon. Besonders geeignet für den Zweck ist dann auch ein Metall mit 14 - 15 Gewichts-% Zr.

Dieses Metall kann weiterhin mittels Kaltumformen verfestigt werden, entsprechend obigen Zahlen. Dann können Festigkeiten gegen 1000 MPa erreicht sein, was den Eigenschaften der alpha-beta Werkstoffe nahekommt. In der Titan-Legierung sind weiterhin als technische Verunreinigungen max. 0,30 Gewichts-% Fe, max. 0,05 Gewichts-% N, max. 0,10 Gewichts-% C und max. 0,015 Gewichts-% H als technische Verunreinigungen enthalten. Zirkon kann je nach Herstellprozess (Iodid - oder Kroll-Prozess) 2 - 3 Gewichts-% Hafnium, ein nicht toxisches Element, enthalten. Die erfindungsgemässe Legierung kann gegebenenfalls als Fremdkomponente bis 0,5 Gewichts-% Hafnium aufweisen.

## Patentansprüche

1. Binäre Titan-Zirkon-Legierung, enthaltend die beiden Legierungsbestandteile Titan und Zirkon und gegebenenfalls bis zu 0.5 Gewichts-% Hafnium (als Verunreinigung von Zirkonium), **dadurch gekennzeichnet, dass** diese Legierung (i) eine binäre Legierung mit einem Anteil an Zirkon von weniger als 25 Gewichts-%, aber mehr als 5 Gewichts-% darstellt, (ii) 0.1 bis 0.3 Gewichts-% Sauerstoff als festigkeitssteigernder Zusatz enthält und (iii) bis maximal 1 Gewichts-% festigkeitssteigern-de Zusätze und technische Verunreinigungen aufweist, wobei (iv) die Komponenten Titan, Zirkon, sowie die gegebenenfalls anwesenden festigkeitssteigernden Zusätze und technische Verunreinigungen zusammen 100 Gewichts-% ergeben.

2. Titan-Zirkon-Legierung nach Anspruch 1, **dadurch gekennzeichnet, dass** der Anteil an Zirkon weniger als 19 Gewichts-% (entsprechend 11 Atom-%), aber mehr als 10 Gewichts-% (entsprechend 5.5 Atom-%) beträgt.

3. Titan-Zirkon-Legierung nach Anspruch 1 oder 2, **dadurch gekennzeichnet, dass** der Anteil an Zirkon 14-15 Gewichts-% beträgt.

4. Titan-Zirkon-Legierung nach einem der Ansprüche 1-3, **dadurch gekennzeichnet, dass** diese vorgängig zu der Verarbeitung zu Implantaten warm geschmiedet und danach kaltverformt wurde.

5. Verwendung der Titan-Zirkonium-Legierung nach einem der Ansprüche 1-4 zur Herstellung von Implantaten für die Zahnchirurgie, Abutments und Elemente für Suprakonstruktionen.

6. Verfahren zur Herstellung der Legierung nach einem der Ansprüche 1-5, **dadurch gekennzeichnet, dass** der Schmiedevorgang bei Temperaturen von über 850°C ausgeführt, die Legierung anschliessend schnell abgekühlt und danach kaltverformt wird.

7. Verfahren zur Herstellung der Legierung nach Anspruch 6, **dadurch gekennzeichnet, dass** der Schmiedevorgang im Bereich der alpha/beta Phasenumwandlung bei 770°C bis 830°C ausgeführt, die Legierung anschliessend schnell abgekühlt und danach kaltverformt wird.

8. Chirurgische Implantate hergestellt aus einer Titan-Zirkon-Legierung nach einem der Ansprüche 1-4.

9. Implantate für die Zahnchirurgie, Abutments und Elemente für Suprakonstruktionen nach Anspruch 8.

## Claims

1. Binary titanium-zirconium alloy, containing the two alloy components titanium and zirconium and possibly up to 0.5% by weight of hafnium (as an impurity of titanium),
**characterised in that** this alloy
(i) represents a binary alloy with a proportion of zirconium of less than 25% by weight but more than 5% by weight,
(ii) contains 0.1 to 0.3 % by weight of oxygen as a strength-increasing additive, and
(iii) has up to a maximum of 1% by weight of strength-increasing additives and technical impurities, wherein
(iv) the components titanium, zirconium, as well as the strength-increasing additives and technical impurities that may be present together make up 100%.

2. Titanium-zirconium alloy in accordance with claim 1, **characterised in that** the proportion of zirconium amounts to less than 19% by weight (corresponding to 11 atomic %), but more than 10% by weight (corresponding to 5.5 atomic %).

3. Titanium-zirconium alloy in accordance with claim 1 or 2, **characterised in that** the proportion of zirconium amounts to 14-15% by weight.

4. Titanium-zirconium alloy in accordance with one of the claims 1-3, **characterised in that** prior to processing into implants, this is hot-forged and then cold-formed.

5. Use of the titanium-zirconium alloy in accordance with one of the claims 1-4 for the manufacture of implants for dental surgery, abutments and elements for supra-constructions.

6. Method for the manufacture of the alloy in accordance with one of the claims 1-5, **characterised in that** the forging process is carried out at temperatures of over 850° C, the alloy is then quickly cooled, and thereafter cold-formed.

7. Method for the manufacture of the alloy in accordance with claim 6, **characterised in that** the forging process is carried out in the region of the alpha/beta phase transition at 770° C to 830°C, the alloy is subsequently quickly cooled, and thereafter cold-formed.

8. Surgical implants manufactured from a titanium-zirconium alloy in accordance with one of the claims 1-4.

9. Implants for dental surgery, abutments and elements for supra-constructions in accordance with claim 8.

## Revendications

1. Alliage binaire titane-zirconium contenant les deux composants d'alliage titane et zirconium et éventuellement jusqu'à 0,5 % en poids de hafnium (comme impureté du zirconium), **caractérisé en ce que** cet alliage
(i) est un alliage binaire avec une proportion de zirconium inférieure à 25 % en poids mais supérieure à 5 % en poids,
(ii) contient 0,1 à 0,3 % en poids d'oxygène comme additif augmentant la solidité, et (iii) comporte au plus 1 % en poids d'additifs augmentant la solidité et d'impuretés techniques, (iv) les composants titane, zirconium ainsi que les additifs augmentant la solidité et les impuretés techniques qui sont éventuellement présents représentant ensemble 100% en poids.

2. Alliage titane-zirconium selon la revendication 1, **caractérisé en ce que** la proportion de zirconium est inférieure à 19 % en poids (correspondant à 11 % atomiques), mais supérieure à 10 % en poids (correspondant à 5,5 % atomiques).

3. Alliage titane-zirconium selon la revendication 1 ou 2, **caractérisé en ce que** la proportion de zirconium est de 14-15 % en poids.

4. Alliage titane-zirconium selon l'une des revendications 1 à 3, **caractérisé en ce que**, avant d'être transformé en implants, il a été forgé à chaud puis a été formé à froid.

5. Utilisation de l'alliage titane-zirconium selon l'une des revendications 1 à 4 pour fabriquer des implants pour la chirurgie dentaire, des aboutements et des éléments pour des supraconstructions.

6. Procédé pour fabriquer l'alliage selon l'une des revendications 1 à 5, **caractérisé en ce que** l'opération de forgeage est effectuée à des températures supérieures à 850°C, l'alliage est ensuite refroidi rapidement puis formé à froid.

7. Procédé pour fabriquer l'alliage selon la revendication 6, **caractérisé en ce que** l'opération de forgeage est effectuée dans la plage de transition de phase alpha/bêta de 770°C à 830°C, l'alliage est ensuite refroidi rapidement puis formé à froid.

8. Implants chirurgicaux fabriqués à partir d'un alliage titane-zirconium selon l'une des revendications 1 à 4.

9. Implants pour la chirurgie dentaire, les aboutements et les éléments pour supraconstructions selon la revendication 8.
